# EUROPÄISCHE PATENTSCHRIFT

(11) **EP 1 017 435 B1**
(45) Veröffentlichungstag und Bekanntmachung des Hinweises auf die Patenterteilung: **15.12.2004**
(21) Anmeldenummer: 98941213.5
(22) Anmeldetag: 16.09.1998
(51) Int. Cl.: A61M 5/20, A61M 5/145

(54) **ABGABEVORRICHTUNG MIT SIGNALGEBER**
DELIVERY DEVICE WITH A SIGNALLING UNIT
DISPOSITIF DE DISTRIBUTION AVEC DONNEUR DE SIGNAUX

(30) Priorität: 25.09.1997 CH 225897
(43) Veröffentlichungstag der Anmeldung: 12.07.2000
(73) Patentinhaber: TecPharma Licensing AG, 3401 Burgdorf (CH)
(72) Erfinder: STECK, Jürg, CH-3422 Kirchberg (CH)
(74) Vertreter: Schwabe - Sandmair - Marx
(86) Internationale Anmeldenummer: PCT/CH1998/000397
(87) Internationale Veröffentlichungsnummer: WO 1999/015214

(56) Entgegenhaltungen:
- WO-A-97/30742
- GB-A- 2 115 495
- US-A- 3 884 228
- US-A- 4 624 658

## Beschreibung

Die Erfindung betrifft eine Vorrichtung zur Ausschüttung eines injizierbaren Produkts mit einem akustischen oder vibrierenden Signalgeber nach dem Oberbegriff von Anspruch 1.

Bei Vorrichtungen, wie sie die Erfindung betrifft, handelt es sich vorzugsweise um Abgabevorrichtungen zur Injektion oder Infusion einer flüssigen Wirkstofflösung, vorzugsweise einer Medikamentenflüssigkeit. Besonders bevorzugt handelt es sich um tragbare Geräte, insbesondere in der Form sogenannter Injektionspens, von denen im folgenden stets auch stellvertretend für andere die Rede ist.

Injektionspens, wie zum Beispiel aus der WO 93/16740 bekannt, aber auch Pumpengeräte, wie zum Beispiel aus EP-B-0 143 895 für Infusion bzw. Infusion/Injektion von Wirkstofflösungen bekannt, weisen ein Gehäuse auf, in dem ein das Produkt enthaltendes Behältnis in einer hierfür vorgesehenen Aufnahme aufgenommen werden kann. Im Behältnis ist ein Kolben verschiebbar aufgenommen. Beim Verschieben des Kolbens in die Vorschubrichtung wird eine Produktdosis aufgrund einer Verdrängung durch den Kolben ausgeschüttet. Hierzu wird das Behältnis, im allgemeinen eine Ampulle, in der Aufnahme derart fixiert, dass ein Abtriebsglied eines Abgabemechanismus den Kolben zur Ausschüttung des Produkts in Vorschubrichtung drückt. Der Abgabemechanismus besteht im wesentlichen aus zwei Elementen, dem bereits erwähnten Abtriebsglied und einer Antriebseinrichtung. Das Abtriebsglied ragt in das Behältnis - auf den Kolben zu - hinein, falls das Behältnis im Gehäuse aufgenommen ist. Mit dem Abtriebsglied steht die Antriebseinrichtung derart in Eingriff, dass durch eine Betätigung der Antriebseinrichtung das Abtriebsglied in Vorschubrichtung schiebbar ist. Bei bekannten Injektionspens mit Spindeltrieben wird die Antriebseinrichtung sowohl durch Drehen, nämlich zum Zweck des Dosierens der mit der nächsten Injektion auszuschüttenden Produktdosis, und auch durch Geradverschiebung in Vorschubrichtung des Kolbens, nämlich durch manuell ausgeübten Druck, betätigt. Denkbar ist aber auch ein Abgabemechanismus dessen Abtriebsglied eine sägezahnartige Struktur aufweist und die Antriebseinrichtung mit entsprechenden Gegenelementen ausgestattet ist. Bei bekannten, mittels Elektromotoren betriebenen, Pumpengeräten mit Spindeltrieben wird die Antriebseinrichtung nur durch Drehen betätigt. Die Drehung bewirkt wiederum einen Vorschub des Abtriebsgliedes, die bei Pumpengeräten jedoch im allgemeinen unmittelbar auf den Kolben übertragen wird.

Die flüssige Wirkstofflösung wird durch eine auf den Injektionspen aufgesetzte Kanüle ausgestossen. Das hintere Ende der Kanüle steht mit der Wirkstofflösung in Verbindung, während das vordere Ende vom Injektionspen abragt und die Haut durchstechen kann. Bei Pumpengeräten befindet sich die Kanüle am der Pumpe gegenüberliegenden Ende eines Infusionsschlauches.

Zur Verabreichung der Wirkstofflösung wird zuerst die Kanüle durch die Haut gestossen und anschliessend durch Betätigung des Abgabemechanismus, sei dies manuell oder motorisch, wird Wirkstofflösung durch die Kanüle in den Körper ausgeschüttet. Die Ausschüttung erfolgt derart, dass bei Betätigung des Abgabemechanismus der Kolben um eine bestimmte Weglänge in Richtung Ampullenauslass verschoben wird, wodurch zuerst eine relativ grosse Menge Wirkstoff ausgeschüttet wird und anschliessend, während einer relativ kurzen Zeit nur noch tropfenweise Wirkstoff durch die Kanüle ausgestossen wird. Bei Wirkstofflösungen, welche in hoher Konzentration verabreicht werden, sind aber diese letzten Tropfen nicht zu vernachlässigen. Der Anwender wird deshalb aufgefordert, nach Betätigung des Abgabemechanismus die Kanüle ungefähr 5 bis 10 Sekunden unter der Haut zu belassen, damit diese letzten Tropfen ebenfalls in den Körper gelangen. Häufig fehlt aber dem Anwender das Zeitgefühl und er zieht die Kanüle zu früh aus dem Körper.

Aus der Schrift WO 97/30742 ist ein Gerät bekannt, welches das oben genannte Problem mit einer elektronischen Anzeige löst. Dies überzeugt nicht, weil der Anwender, insbesondere bei Insulinabgabegeräten, häufig unter Augenerkrankungen leidet und visuelle Anzeigen deshalb eher unglücklich sind. Darüber hinaus sind visuelle Anzeigen bei Geräten im Gebrauch ungünstig, weil der Gebrauch des Gerätes derart erfolgen muss, dass der Anwender Sichtkontakt mit der Anzeigefläche des Gerätes hat.

Hier will die Erfindung Abhilfe verschaffen. Der Erfindung liegt die Aufgabe zugrunde, eine Vorrichtung zur Abgabe einer Produktdosis anzugeben, welche den Anwender mittels einem akustischen Signal oder mittels Vibration auffordert die Kanüle aus dem Körper zu entfernen.

Die Erfindung löst die gestellte Aufgabe mit einer Vorrichtung, welche die Merkmale des Anspruchs 1 aufweist.

Die durch die Erfindung erreichten Vorteile sind im wesentlichen darin zu sehen, dass durch das akustische Signal oder.die Vibration der Anwender auf einfache Art und Weise darauf aufmerksam gemacht wird, dass die letzten Tropfen einer Injektion/Infusion in den Körper gelangt sind und daher die Kanüle aus dem Körper entfernt werden kann.

Bevorzugte Ausführungsbeispiele der Erfindung werden nachfolgend anhand von Zeichnungen erläutert.

Es zeigen:
- Fig. 1: Eine erfindungsgemässe Abgabevorrichtung mit einem Signalgeber
- Fig. 2-4: alternative Ausführungsbeispiele für den Signalgeber und die Abgabevorrichtung

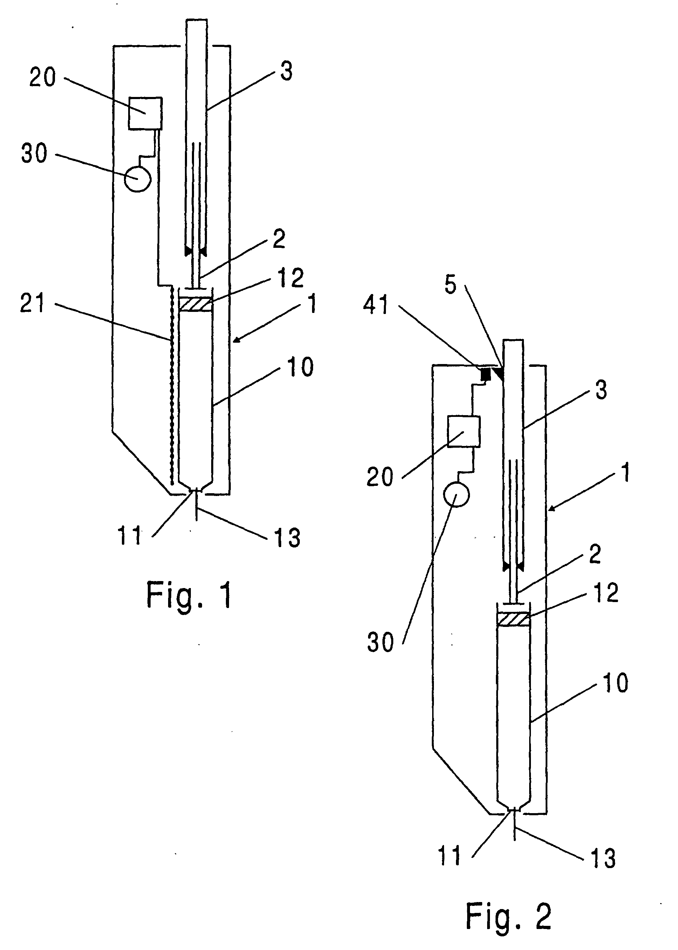

Wie in der Figur 1 dargestellt, weist die erfindungsgemässe Abgabevorrichtung ein Gehäuse 1 auf, in welchem eine Ampulle 10 gelagert ist. In der Ampulle wird eine Wirkstofflösung zwischen einem vorderen Auslass 11, meist als Durchstechmembran ausgebildet, und einem gleitbeweglichen Kolben 12 gelagert.

Der Abgabemechanismus besitzt ein Abtriebsglied 2 und eine damit in Eingriff stehende Antriebseinrichtung 3. Durch eine Betätigung der Antriebseinrichtung 3 ist das Abtriebsglied 2 in Vorschubrichtung schiebbar. Die Einstellung der zu verabreichenden Produktdosis erfolgt durch Vergrösserung der Gesamtweglänge von Antriebseinrichtung 3 und Abtriebsglied 2. Um die gewählte Vergrösserung der Gesamtweglänge von Antriebseinrichtung 3 und Abtriebsglied 2 wird der Kolben 12 der Ampulle 10 in Richtung Ampullenauslass 11 geschoben, so dass eine Produktdosis aufgrund einer Verdrängung durch eine, beim Ampullenauslass 11 aufgesetzte, Kanüle 13 erfolgt.

Innerhalb des Gerätes 1 befindet sich eine Vorrichtung 30, welche mit einer zeitlichen Verzögerung ein akustisches oder vibrierendes Signal abgeben soll, wenn der Kolben 12 die gesamte gewünschte Weglänge zurückgelegt hat und damit die jeweilige Ausschüttung der Produktdosis erfolgt ist. Um dieses Problem zu lösen bieten sich diverse Lösungen an. So kann mittels Sensoren 21, welche entlang der Ampulle angeordnet werden, jeweils registriert werden, wenn der Kolben 12 nach einer gewissen Vorwärtsbewegung zum Stillstand gekommen ist (Fig. 1). Ist dieser Stillstand registriert worden, wird ein Zeitablaufsystem 20 - vorzugsweise eine mechanische oder eine elektronische Steuerung - aktiviert und nach dessen Ablauf vom akustischen oder vibrierenden Signalgeber 30 ein Signal abgegeben, welches den Anwender darüber informiert, dass seit der Beendigung der Verschiebung des Kolbens 12 in Richtung Ampullenauslass 11 eine bestimmte Zeiteinheit abgelaufen ist. Als ideale zeitliche Verzögerung zwischen letzter Bewegung des Stopfens und akustischem oder vibrierenden Signal gelten 5 bis 10 Sekunden.

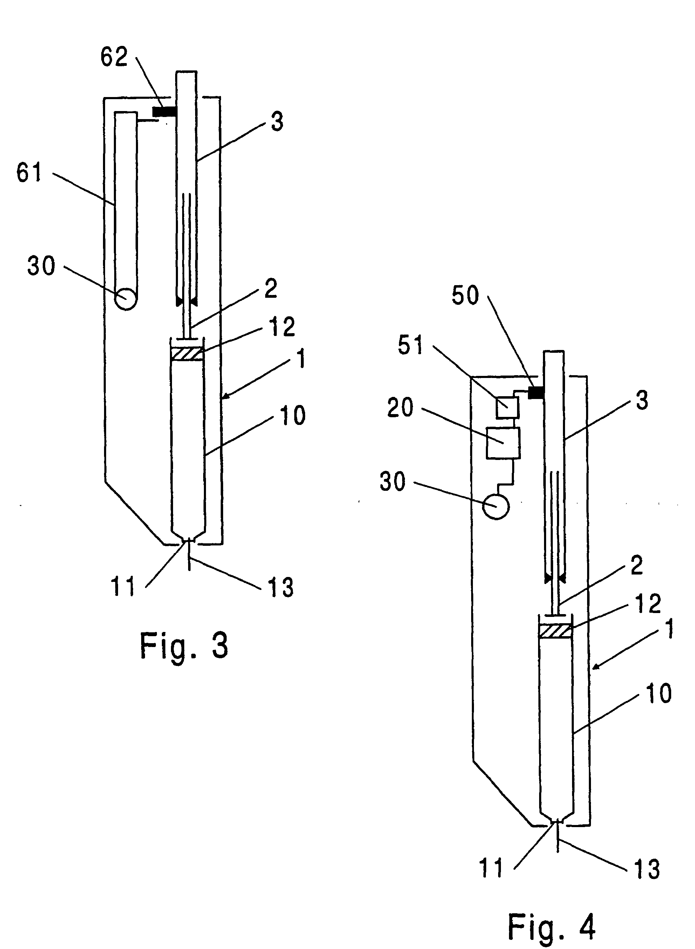

Bei manuell angetriebenen Abgabevorrichtungen können an der Antriebseinrichtung 3 Einrastmittel 5 angeordnet werden, welche nach Betätigung im Gehäuse 1 einrasten (Fig. 2). Bei der Einrastung werden Mittel 41 aktiviert, welche einen Impuls an das Zeitablaufsystem 20 geben und nach dessen Ablauf wird vom akustischen oder vibrierenden Signalgeber 30 ein Signal abgegeben. Bei den obengenannten Mitteln 41 kann es sich um Sensoren handeln, welche das Einrasten erkennen oder um einen Schalter, welcher bei der Einrastbewegung aktiviert wird. Ebenfalls denkbar ist der Einsatz eines Aufziehweckers 61, wobei bei Betätigung der Antriebseinrichtung 3 über ein Verbindungselement 62 gleichzeitig der Wecker 61 aufgezogen wird (Fig. 3). Der Signalgeber 30 wird dabei Teil des Weckers 61 sein.

Wie in Fig. 4 dargestellt, kann bei motorbetriebenen Abgabevorrichtungen eine Motorsteuerung 51 nebst dem Stoppbefehl an den Motor 50 ein Impuls an das Zeitablaufsystem 20 abgeben, wobei nach dessen Ablauf vom akustischen oder vibrierenden Signalgeber 30 ein Signal abgegeben wird.

Der Signalgeber kann auch ausserhalb der Abgabevorrichtung angeordnet sein und mittels elektrischer Verbindung oder mittels Funkverbindung aktiviert werden.

## Patentansprüche

1. Tragbare Vorrichtung zur Ausschüttung eines flüssigen Produkts per Injektion oder Infusion, die Vorrichtung umfassend:
a) ein Gehäuse ( 1 ),
b) einen Flüssigkeitsbehälter (10) für das Produkt,
c) einen manuell oder motorisch betriebenen Abgabemechanismus (2, 3), mittels dem das Produkt aus dem Flüssigkeitsbehälter (10) ausschüttbar ist,
d) einen Signalgeber (30) zur Abgabe eines akustischen oder vibratorischen Signals,
e) ein Zeitablaufsystem (20)
f) und ein mit dem Zeitablaufsystem (20) verbundenes Aktivierungsmittel (21, 41, 51, 61) zur Aktivierung des Signalgebers (30), wobei das Aktivierungsmittel (21, 41, 51, 61) im Falle einer Betätigung des Abgabemechanismus (2, 3) spätestens bei Beendigung der Betätigung das Zeitablaufsystem (20) aktiviert,
**dadurch gekennzeichnet, dass**
g) der Signalgeber (30) derart gestaltet ist, dass dieser nach der Aktivierung des Zeitablaufsystems (20) das Signal verzögert erst nach Ablauf des Zeitablaufsystems (20) abgibt.

2. Vorrichtung nach Anspruch 1, **dadurch gekennzeichnet, dass** das Aktivierungsmittel (61) ein mechanisches Mittel ist.

3. Vorrichtung nach Anspruch 1, **dadurch gekennzeichnet, dass** das Aktivierungsmittel (41) ein elektrisches Mittel, vorzugsweise ein Schalter, ist.

4. Vorrichtung nach Anspruch 1, **dadurch gekennzeichnet, dass** das Aktivierungsmittel (21) ein elektronisches Mittel, vorzugsweise ein Sensor, ist.

5. Vorrichtung nach einem der vorhergehenden Ansprüche, **dadurch gekennzeichnet, dass** das Zeitablaufsystem (20) eine mechanische oder elektronische Steuerung ist.

6. Vorrichtung nach Anspruch 1, **dadurch gekennzeichnet, dass** an dem Abgabemechanismus (2, 3) Einrastmittel (5) angeordnet sind, die bei der Beendigung der Betätigung des Abgabemechanismus (2, 3) in ein Gegenelement des Gehäuses (1) einrasten.

7. Vorrichtung nach dem vorhergehenden Anspruch, **dadurch gekennzeichnet, dass** die Einrastmittel (5) auf einen das Aktivierungsmittel (41) bildenden Schalter wirken, wodurch das Zeitablaufsystem (20) aktiviert wird.

8. Vorrichtung nach einem der vorhergehenden Ansprüche, **dadurch gekennzeichnet, dass** mindestens ein das Aktivierungsmittel (21) bildender Sensor die Beendigung der Betätigung des Abgabemechanismus (2, 3) feststellt, wodurch das Zeitablaufsystem (20) aktiviert wird.

9. Vorrichtung nach Anspruch 1, **dadurch gekennzeichnet, dass** ein mechanischer Verzögerungsschalter (30, 61) das Aktivierungsmittel und das Zeitablaufsystem bildet, dass die Vorrichtung ein den Abgabemechanismus (2, 3) mit dem Verzögerungsschalter (61) verbindendes Verbindungselement (62) aufweist und dass das Aktivierungsmittel bei der Betätigung des Abgabemechanismus (2, 3) das Zeitablaufsystem mittels des Verbindungselements (62) aktiviert.

10. Vorrichtung nach dem vorhergehenden Anspruch, **dadurch gekennzeichnet, dass** ein aufziehbarer Wecker (61, 30) den Verzögerungsschalter bildet.

11. Vorrichtung nach Anspruch 1, **dadurch gekennzeichnet, dass** der Abgabemechanismus (2, 3) einen Motor (50) mit einer das Aktivierungsmittel bildenden Steuerung (51) umfasst, wobei die Steuerung (51) einen Stoppbefehl für den Motor (50) erzeugt und in zeitlicher Abhängigkeit von der Erzeugung des Stoppbefehls das Zeitablaufsystem (20) aktiviert.

12. Vorrichtung nach einem der vorhergehenden Ansprüche, **dadurch gekennzeichnet, dass** der Flüssigkeitsbehälter eine mit einem Kolben (12) ausgestattete Ampulle (10) ist, in der die zu verabreichende Flüssigkeit zwischen dem Kolben (12) und einer Auslassöffnung (11) lagert.

13. Vorrichtung nach dem vorhergehenden Anspruch, **dadurch gekennzeichnet, dass** der Abgabemechanismus ein auf den Kolben (12) wirkendes, stabförmiges Abtriebsglied (2) mit einem strukturierten äußeren Mantel und eine Antriebseinrichtung (3) umfasst, die teilweise um das Abtriebsglied (2) angeordnet ist und einen zu dem äußeren Mantel des Abtriebsglieds (2) passenden inneren Mantel aufweist.

14. Vorrichtung nach Anspruch 12, **dadurch gekennzeichnet, dass** der Abgabemechanismus ein auf den Kolben wirkendes, stabförmiges Abtriebsglied mit einem strukturierten inneren Mantel und eine Antriebseinrichtung umfasst, die teilweise innerhalb des Abtriebsglieds angeordnet ist und einen zu dem inneren Mantel des Abtriebsglieds passenden äußeren Mantel aufweist.

15. Vorrichtung nach einem der drei vorhergehenden Ansprüche, **dadurch gekennzeichnet, dass** mindestens ein das Aktivierungsmittel bildender Sensor (21) die Beendigung der Bewegung des Kolbens (12) feststellt und das Zeitablaufsystem (20) aktiviert.

16. Vorrichtung nach einem der vorhergehenden Ansprüche, **dadurch gekennzeichnet, dass** der Signalgeber (30) innerhalb des Gehäuses (1) angeordnet ist.

17. Vorrichtung nach einem der Ansprüche 1 bis 15, **dadurch gekennzeichnet, dass** der Signalgeber (30) außerhalb des Gehäuses (1) angeordnet ist.

## Claims

1. A portable device for delivering a fluid product by injection or infusion, said device comprising:
a) a casing (1);
b) a fluid container (10) for the product;
c) a manual or motor-driven dispensing mechanism (2, 3), by means of which the product can be delivered from the fluid container (10);
d) a signal transmitter (30) for emitting an acoustic or vibration signal;
e) a time lapse system (20);
f) and an activating means (21, 41, 51, 61) connected to the time lapse system (20), for activating the signal transmitter (30), wherein when the dispensing mechanism (2, 3) is operated, the activating means (21, 41, 51, 61) activates the time lapse system (20) at the latest at the end of operation,
**characterised in that**
g) the signal transmitter (30) is configured such that, once the time lapse system (20) has been activated, the signal transmitter (30) emits the signal delayed and not until after the time lapse system (20) has lapsed.

2. The device as set forth in claim 1, **characterised in that** the activating means (61) is a mechanical means.

3. The device as set forth in claim 1, **characterised in that** the activating means (41) is an electrical means, preferably a switch.

4. The device as set forth in claim 1, **characterised in that** the activating means (21) is an electronic means, preferably a sensor.

5. The device as set forth in any one of the preceding claims, **characterised in that** the time lapse system (20) is a mechanical or electronic control system.

6. The device as set forth in claim 1, **characterised in that** latching means (5) are arranged on the dispensing mechanism (2, 3) which latch into a counter element of the casing (1) at the end of operating the dispensing mechanism (2, 3).

7. The device as set forth in the preceding claim, **characterised in that** the latching means (5) act on a switch forming the activating means (41), whereby the time lapse system (20) is activated.

8. The device as set forth in any one of the preceding claims, **characterised in that** at least one sensor forming the activating means (21) establishes the end of operating the dispensing mechanism (2, 3), whereby the time lapse system (20) is activated.

9. The device as set forth in claim 1, **characterised in that**: a mechanical delay switch (30, 61) forms the activating means and the time lapse system; the device comprises a connecting element (62) which connects the dispensing mechanism (2, 3) to the delay switch (30, 61); and the activating means activates the time lapse system by means of the connecting element (62), when the dispensing mechanism (2, 3) is operated.

10. The device as set forth in the preceding claim, **characterised in that** an alarm which can be wound up (61, 30) forms the delay switch.

11. The device as set forth in claim 1, **characterised in that** the dispensing mechanism (2, 3) comprises a motor (50) including a control system (51) forming the activating means, wherein the control system (51) generates a stop command for the motor (50) and activates the time lapse system (20) in chronological dependence upon generating the stop command.

12. The device as set forth in any one of the preceding claims, **characterised in that** the fluid container is an ampoule (10) fitted with a piston (12), in which the fluid to be administered is stored between the piston (12) and an outlet opening (11).

13. The device as set forth in the preceding claim, **characterised in that** the dispensing mechanism comprises a rod-shaped driven member (2), acting on the piston (12) and comprising a structured outer surface, and a drive means (3) which is partially arranged around the driven member (2) and exhibits an inner surface which fits the outer surface of the driven member (2).

14. The device as set forth in claim 12, **characterised in that** the dispensing mechanism comprises a rod-shaped driven member, acting on the piston and comprising a structured inner surface, and a drive means which is partially arranged inside the driven member and exhibits an outer surface which fits the inner surface of the driven member.

15. The device as set forth in any one of the preceding three claims, **characterised in that** at least one sensor (21) forming the activating means establishes the end of operating the piston (12) and activates the time lapse system (20).

16. The device as set forth in any one of the preceding claims, **characterised in that** the signal transmitter (30) is arranged inside the casing (1).

17. The device as set forth in any one of claims 1 to 15, **characterised in that** the signal transmitter (30) is arranged outside the casing (1).

## Revendications

1. Dispositif portable de distribution d'un produit liquide par injection ou par perfusion, comportant :
a) un boîtier ( 1 ),
b) un récipient de liquide (10) pour le produit,
c) un mécanisme de distribution (2, 3) manuel ou motorisé, au moyen duquel le produit peut être distribué hors du récipient de liquide (10),
d) un émetteur de signaux (30), fournissant un signal acoustique ou vibratoire,
e) un système à déroulement temporel (20),
f) un moyen d'activation (21, 41, 51, 61) relié au système à déroulement temporel (20) pour activer l'émetteur de signaux (30), le moyen d'activation (21, 41, 51, 61) activant le système à déroulement temporel en cas d'actionnement du mécanisme de distribution (2, 3), au plus tard lorsque l'actionnement est terminé,
**caractérisé en ce que**
g) l'émetteur de signaux (30) est réalisé de telle sorte qu'après l'activation du système à déroulement temporel (20), celui-ci fournit le signal retardé seulement après écoulement du système à déroulement temporel (20).

2. Dispositif selon la revendication 1, **caractérisé en ce que** le moyen d'activation (61) est un moyen mécanique.

3. Dispositif selon la revendication 1, **caractérisé en ce que** le moyen d'activation (41) est un moyen électrique, ' de préférence un commutateur.

4. Dispositif selon la revendication 1, **caractérisé en ce que** le moyen d'activation (21) est un moyen électronique, de préférence un capteur.

5. Dispositif selon l'une des revendications précédentes, **caractérisé en ce que** le système à déroulement temporel (20) est une commande mécanique ou électronique.

6. Dispositif selon la revendication 1, **caractérisé en ce que** sur le moyen de distribution (2, 3) sont agencés des moyens d'enclenchement (5) qui s'enclenchent dans un élément antagoniste du boîtier (1) lors de la fin de l'actionnement du mécanisme de distribution (2,3).

7. Dispositif selon la revendication précédente, **caractérisé en ce que** les moyens d'enclenchement (5) agissent sur un commutateur formant le moyen d'activation (41), ce qui active le système à déroulement temporel (20).

8. Dispositif selon l'une des revendications précédentes, **caractérisé en ce qu'**au moins un capteur formant le moyen d'activation (21) constate la terminaison de l'actionnement du mécanisme de distribution (2, 3), grâce à quoi le système à déroulement temporel (20) est activé.

9. Dispositif selon la revendication 1, **caractérisé en ce qu'**un commutateur à temporisation (30, 61) mécanique forme le moyen d'activation et le système à déroulement temporel, **en ce que** le dispositif présente un élément de connexion (62) reliant le mécanisme de distribution (2, 3) au commutateur à temporisation (61), et **en ce que** le moyen d'activation active lors de l'actionnement du mécanisme de distribution (2, 3) le système à déroulement temporel au moyen de l'élément de connexion (62).

10. Dispositif selon l'une des revendications précédentes, **caractérisé en ce qu'**un réveil (61, 30) susceptible d'être remonté forme le commutateur à temporisation.

11. Dispositif selon la revendication 1, **caractérisé en ce que** le mécanisme de distribution (2, 3) comprend un moteur (50) avec une commande (51) formant le moyen d'activation, la commande (51) engendrant une instruction d'arrêt pour le moteur (50) et activant le système à déroulement temporel (20) en fonction temporelle de la génération de l'instruction d'arrêt.

12. Dispositif selon l'une des revendications précédentes, **caractérisé en ce que** le récipient de liquide est une ampoule (10) équipée d'un piston (12) dans laquelle le liquide à administrer est logé entre le piston (12) et un orifice de sortie (11).

13. Dispositif selon la revendication précédente, **caractérisé en ce que** le mécanisme de distribution comprend un organe mené (2) en forme de tige agissant sur le piston (12), avec une enveloppe extérieure structurée, et un dispositif d'entraînement (3) qui est agencé partiellement autour de l'organe mené (2) et qui présente une enveloppe intérieure adaptée à l'enveloppe extérieure de l'organe mené (2).

14. Dispositif selon la revendication 12, **caractérisé en ce que** le mécanisme de distribution comprend un organe mené (2) en forme de tige agissant sur le piston (12), avec une enveloppe intérieure structurée, et un dispositif d'entraînement (3) qui est agencé partiellement à l'intérieur de l'organe mené (2) et qui présente une enveloppe extérieure adaptée à l'enveloppe intérieure de l'organe mené (2).

15. Dispositif selon l'une des trois revendications précédentes, **caractérisé en ce qu'**au moins un capteur (21) formant le moyen d'activation constate que le mouvement du piston (12) est terminé et active le système à déroulement temporel (20).

16. Dispositif selon l'une des revendications précédentes, **caractérisé en ce que** l'émetteur de signaux (30) est agencé à l'intérieur du boîtier (1).

17. Dispositif selon l'une des revendications 1 à 15, **caractérisé en ce que** ce que l'émetteur de signaux (30) est agencé à l'extérieur du boîtier (1).
